(19) Europäisches Patentamt
European Patent Office
Office européen des brevets

(11) Veröffentlichungsnummer: **0 535 463 A1**

# (12) EUROPÄISCHE PATENTANMELDUNG

(21) Anmeldenummer: **92115970.3**

(22) Anmeldetag: **18.09.92**

(51) Int. Cl.[5]: **C07D 403/10**, A61K 31/415, A61K 31/41

(30) Priorität: **01.10.91 DE 4132631**

(43) Veröffentlichungstag der Anmeldung: **07.04.93 Patentblatt 93/14**

(84) Benannte Vertragsstaaten: **AT BE CH DE DK ES FR GB GR IE IT LI LU MC NL PT SE**

(71) Anmelder: **BAYER AG**

**W-5090 Leverkusen 1 Bayerwerk(DE)**

(72) Erfinder: **Krämer, Thomas, Dr.**
**In den Birken 92a**
**W-5600 Wuppertal 1(DE)**
Erfinder: **Dressel, Jürgen, Ph.Dr.**
**Claudiusweg 9**
**W-5600 Wuppertal 1(DE)**
Erfinder: **Hanko, Rudolf, Dr.**
**Waldsaum 25**
**W-4300 Essen(DE)**
Erfinder: **Hübsch, Walter, Dr.**
**Wildsteig 22**
**W-5600 Wuppertal 1(DE)**
Erfinder: **Müller, Ulrich, Dr.**
**Claudiusweg 5**
**W-5600 Wuppertal 1(DE)**
Erfinder: **Müller-Gliemann, Matthias, Dr.**
**Claudiusweg 5**
**W-5600 Wuppertal 1(DE)**
Erfinder: **Beuck, Martin, Dr.**
**Trills 7**
**W-4006 Erkrath 2(DE)**
Erfinder: **Kazda, Stanislav, Prof. Dr.**
**Gellertweg 18**
**W-5600 Wuppertal 1(DE)**
Erfinder: **Stasch, Johannes-Peter, Dr.**
**Schneewittchenweg 37**
**W-5600 Wuppertal 1(DE)**
Erfinder: **Knorr, Andreas, Dr.**
**Trillser Graben 10**
**W-4006 Erkrath 2(DE)**
Erfinder: **Wohlfeil, Stefan, Dr.**
**Tucherweg 25**
**W-4010 Hilden(DE)**

## (54) Imidazolyl-propensäurederivate als Angiotensin II Antagonisten.

(57) Imidazolyl-propensäurederivate der allgemeinen Formel

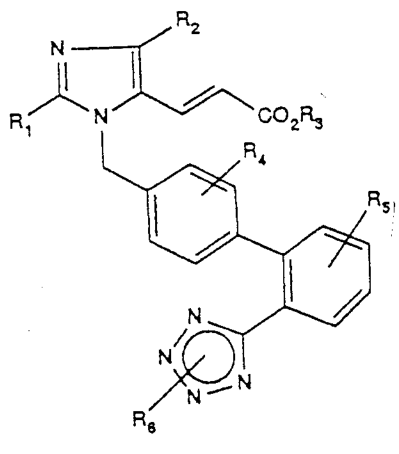

EP 0 535 463 A1

können durch Umsetzung von Aldehyden mit Phosphonoessigsäureestern hergestellt werden. Die Imidazolylpropensäurederivate eignen sich als Wirkstoffe in Arzneimitteln, insbesondere in blutdrucksenkenden und antiatherosklerotischen Arzneimitteln.

Die Erfindung betrifft Imidazolyl-propensäurederivate, ein Verfahren zu ihrer Herstellung und ihre Verwendung als blutdrucksenkende und anti-atherosklerostische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdruckerhöhenden Oktapeptid Angiotensin II abgebaut wird. Die verschiedenen Effekte des Arigiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

In den Publikationen EP 324 377, EP 403 158 und EP 403 159 werden Imidazole beschrieben, bei denen der Bedeutungsumfang der Position 2 (siehe erfindungsgemäße Verbindungen $-CH=CH-CO_2R^3$) Propensäuren und -ester erfaßt und der endständige Phenylring durch einen Tetrazolring substituiert ist, ohne jedoch einen Hinweis auf einen konkreten Stoffvertreter dieses Substitutionsmusters zu geben.

Es wurden nun substituierte Imidazole, deren 2-Position durch eine Propensäure bzw. -estergruppe und deren endständiger Phenylringe durch einen Tetrazolring substituiert sind, mit überraschenderweise guter A-II-antagonistischer Wirkung gefunden.

Die Erfindung betrifft jetzt Imidazolyl-propensäurederivate der allgemeinen Formel (I)

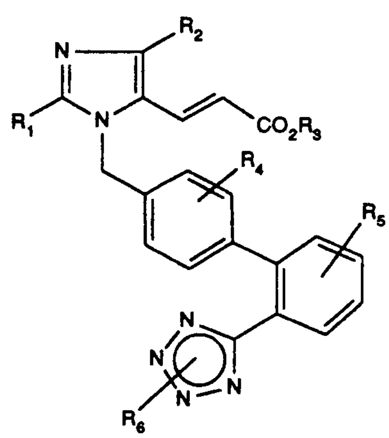

(I),

in welcher

| | |
|---|---|
| $R_1$ | für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder<br>für Cycloalkyl mit 3 bis 8 Kohlenstoffatomen steht, |
| $R_2$ | für Wasserstoff, Halogen, Hydroxy, Nitro, Cyano, Trifluormethyl, Trifluormethoxy oder für Pentafluorethyl steht, oder<br>für geradkettiges oder verzweigtes Alkyl mit bis zu 6 Kohlenstoffatomen steht, oder<br>für Aryl mit 6 bis 10 Kohlenstoffatomen steht, |
| $R_3$ | für Wasserstoff, geradkettiges oder verzweigtes Alkyl mit bis zu 8 Kohlenstoffatomen oder Phenyl steht, |
| $R_4$ und $R_5$ | gleich oder verschieden sind und<br>für Wasserstoff, Halogen, Cyano, Nitro, Trifluormethyl, Hydroxy, Trifluormethoxy oder für geradkettiges oder verzweigtes Alkyl oder Alkoxy mit jeweils bis zu 6 Kohlenstoffatomen stehen, |
| $R_6$ | für Wasserstoff oder für geradkettiges oder verzweigtes Alkyl mit bis zu 6 Kohlenstoffatomen steht |

und deren Salze.

Die erfindungsgemäßen Imidazolyl-propensäurederivate können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Imidazolyl-propensäurederivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di-bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher

$R_1$ für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind,
für Cyclopropyl, Cyclopentyl oder Cyclohexyl steht,

$R_2$ für Wasserstoff, Fluor, Chlor, Brom, Iod, Trifluormethyl, Trifluormethoxy, Pentafluorethyl, Phenyl oder für geradkettiges oder verzweigtes Alkyl mit bis zu 6 Kohlenstoffatomen steht,

$R_3$ für Wasserstoff oder für geradkettiges oder verzweigtes Alkyl mit bis zu 6 Kohlenstoffatomen steht,

$R_4$ und $R_5$ gleich oder verschieden sind und
für Wasserstoff, Fluor, Chlor, Brom, Trifluormethyl, Trifluormethoxy oder für geradkettiges oder verzweigtes Alkyl mit bis zu 4 Kohlenstoffatomen stehen,

$R_6$ für Wasserstoff oder für geradkettiges oder verzweigtes Alkyl mit bis zu 4 Kohlenstoffatomen steht

und deren Salze.

Besonders bevorzugt sind Verbindungen der allgemeinen Formel (I),
in welcher

$R_1$ für geradkettiges oder verzweigtes Alkyl oder Alkenyl mit jeweils bis zu 4 Kohlenstoffatomen oder Cyclopropyl steht,

$R_2$ für Wasserstoff, Fluor, Chlor, Brom, Jod, Trifluormethyl, Pentafluorethyl, Phenyl oder für geradkettiges oder verzweigtes Alkyl mit bis zu 4 Kohlenstoffatomen steht,

$R_3$ für Wasserstoff oder für geradkettiges oder verzweigtes Alkyl mit bis zu 4 Kohlenstoffatomen steht,

$R_4$ und $R_5$ gleich oder verschieden sind und
für Wasserstoff, Fluor, Chlor oder Brom stehen,

$R_6$ für Wasserstoff, Methyl, Ethyl, Isopropyl oder Propyl steht

und deren Salze.

Ganz besonders bevorzugt sind Verbindungen der allgemeinen Formel (I),
in welcher

$R_1$ für geradkettiges oder verzweigtes Alkyl oder Alkenyl mit jeweils bis zu 4 Kohlenstoffatomen steht,

$R_2$ für Wasserstoff, Fluor, Chlor, Iod, Trifluormethyl oder Pentafluorethyl steht,

$R_3$ für Wasserstoff, Methyl oder Ethyl steht

und

$R_4$, $R_5$ und $R_6$ für Wasserstoff stehen

und deren Salze.

Außerdem wurde ein Verfahren zur Herstellung der erfindungsgemäßen Verbindungen der allgemeinen Formel (I), und deren Salze gefunden, dadurch gekennzeichnet, daß man Aldehyde der allgemeinen Formel (II)

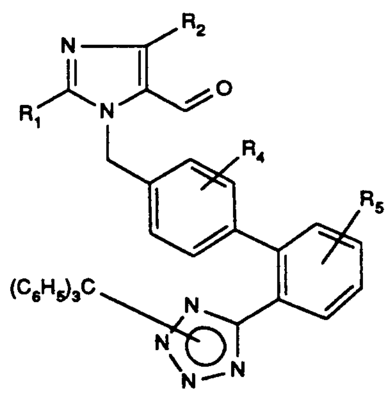

(II),

in welcher $R_1$, $R_2$, $R_4$ und $R_5$ die oben angegebene Bedeutung haben, mit Phosphonoessigsäureestern der allgemeinen Formel (III)

$(R^7O)_2$-P(O)-$CH_2$-$CO_2R^7$ (III),

in welcher

$R_7$ die oben angegebene Bedeutung von $R_3$ hat, aber nicht für Wasserstoff steht,

in inerten Lösemitteln, in Anwesenheit einer Base zu den Verbindungen der allgemeinen Formel (IV)

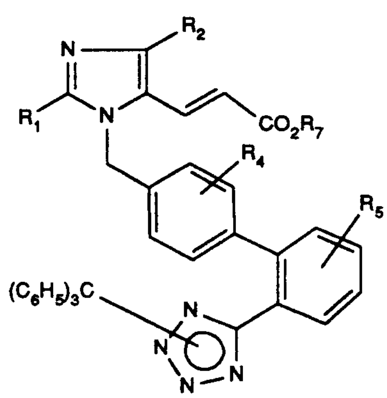

(IV),

in welcher $R_1$, $R_2$, $R_4$, $R_5$ und $R_7$ die oben angegebene Bedeutung haben, umsetzt und anschließend die Tritylgruppe mit Säuren abspaltet, und im Fall der Säuren ($R_3$ = H) die Ester verseift und im Fall, daß $R_6$ nicht für Wasserstoff steht, alkyliert.

Das erfindungsgemäße Verfahren kann durch folgendes Formelschema beispielhaft erläutert werden:

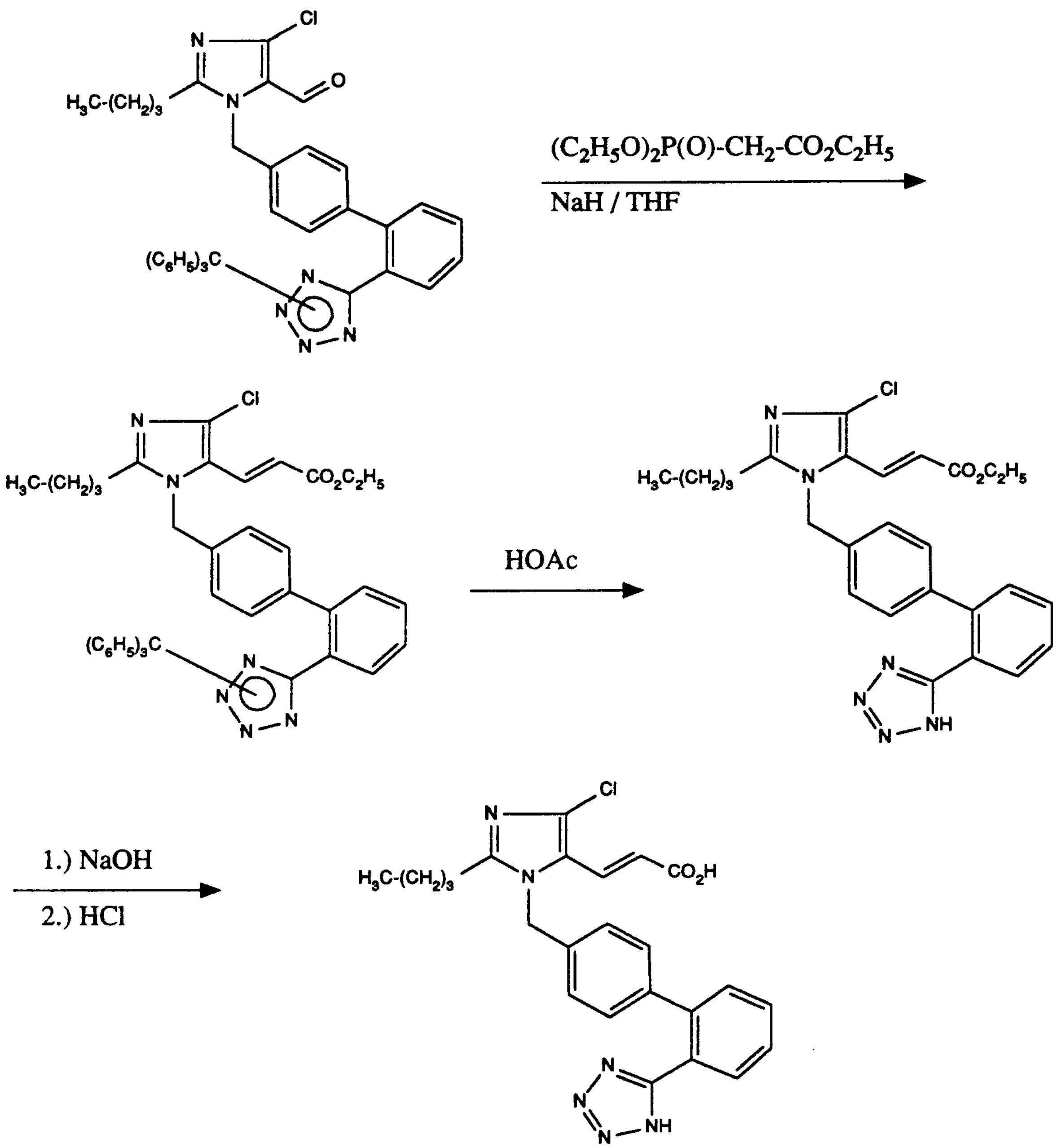


Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt ist Natriumhydrid.

Im allgemeinen setzt man die Base in einer Menge von 0,05 mol bis 10 mol, bevorzugt von 1 mol bis 2 mol bezogen auf 1 mol der Verbindung der Formel (III) ein.

Das erfindungsgemäße Verfahren wird im allgemeinen in einem Temperaturbereich von -30°C bis +100°C, bevorzugt von -10°C bis +60°C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Säuren für die Abspaltung der Triphenylmethylgruppe eignen sich im allgemeinen organische, gegebenenfalls halogenierte $C_1$-$C_6$-Carbonsäuren. Bevorzugt sind Eisessig und Trifluoressigsäure.

Die Abspaltung erfolgt im allgemeinen in einem Temperaturbereich von 0°C bis +120°C, vorzugsweise von +20°C bis +100°C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden. Im Fall der basischen Heterocyclen können durch das Behandeln der Lösungen der Carboxylate mit den oben aufgeführten Säuren auch die Salze der Heterocyclen mit den anorganischen Säuren gewonnen werden.

Die Alkylierung erfolgt im allgemeinen mit Alkylierungsmitteln wie beispielsweise ($C_1$-$C_6$)-Alkylhalogeniden, Sulfonsäureestern oder substituierten oder unsubstituierten ($C_1$-$C_6$)-Dialkyl- oder ($C_1$-$C_6$)-Diarylsulfonate, vorzugsweise Methyljodid oder Dimethylsulfat.

Die Alkylierung erfolgt im allgemeinen in einem der oben aufgeführten Lösemitteln, vorzugsweise in Dimethylformamid in einem Temperaturbereich von 0°C bis +70°C, vorzugsweise von 0°C bis +30°C und Normaldruck.

Die Aldehyde der allgemeinen Formel (II) sind an sich bekannt oder können nach üblicher Methode hergestellt werden [vgl. PCT-Anmeldung WO 91/00277].

Ebenso sind die Phosphonoessigsäureester der allgemeinen Formel (III) bekannt [vgl. Beilstein 4 (1) 573].

Die Verbindungen der allgemeinen Formel (IV) sind als konkrete Stoffvertreter neu und können dann beispielsweise nach dem oben angegebenen Verfahren hergestellt werden.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen Imidazolyl-propensäuren und -derivate zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimittel zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

Agonisten und ihre Standardkonzentrationen (Applikationsvolumen pro Einzelgabe = 100µl):

| | | |
|---|---|---|
| KCl | 22,7;32,7;42,7;52,7 | mmol/l |
| L-Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oderin hohen Konzentrationen nur schwach gehemmt.

Tabelle A:

Hemmung der Gefäßkontraktion an isolierten Aortenringen von Kaninchen in vitro

$IC_{50}$ (g/ml) gegen Kontraktionen, induziert durch:

| Bsp.Nr.: | AII |
|---|---|
| 1 | 2.5 x $10^{-8}$ g/ml |
| 2 | 2-1 x $10^{-8}$ g/ml |

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.
Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde Rind

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), $^3$H-Angiotensin II(3-5 nM), Test-Pufferlösung (50 mM Tris, pH 7,2, 5 mM $MgCl_2$, 0,25% BSA) sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins

mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der totalen Bindung des Radioliganden bewirkt).

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 24-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert. Danach werden die Zellen durch Serumentzug für 2 - 3 Tage synchronisiert und sodann mit AII, Serum oder anderen Faktoren zum Wachstum angeregt. Gleichzeitig werden Testverbindungen zugesetzt. Nach 16 - 20 Stunden wird 1 µCi $^3$H-Thymidin zugefügt und nach weiteren 4 Stunden der Einbau dieser Substanz in die TCA-präzipitierbare DNA der Zellen bestimmt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Köpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

Ausgangsverbindungen

Beispiel I

2-n-Butyl-1-[(2'-(N-triphenylmethyl-tetrazol-5-yl)biphenyl-4-yl)methyl]-1H-imidazol-5-carboxaldehyd

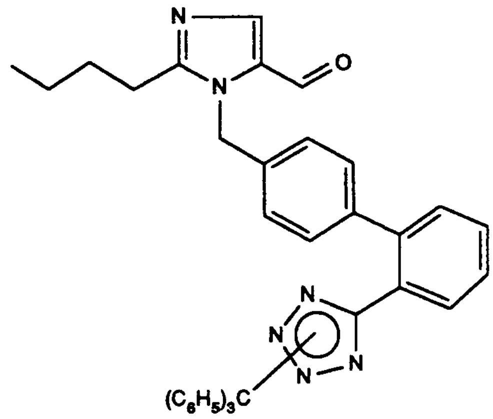

12,0 g (18,1 mmol) 2-n-Butyl-4-chlor-1-(2'-(N-triphenylmethyltetrazol-5-yl)-biphenyl-4-yl)methyl]-1H-imidazol-5-carboxaldehyd in 150 ml Methanol werden bei 25 °C in Gegenwart von 1,2 g Palladium auf Aktivkohle (5 %ig) und 2,46 g (18,1 mmol) Natriumacetat-Trihydrat 1,5 h bei ca. 3 bar Wasserstoffdruck hydriert. Danach wird vom Katalysator abfiltriert, eingeengt und der Rückstand über Kieselgel 60 mit Essigester/Petrolether(1:1) chromatographiert.
Ausbeute: 3,85 g (34 % der Theorie)
$R_f$ = 0,41 (Essigester/Petrolether = 1:1).

Beispiel II

(E)-3-[2-n-Butyl-4-chlor-1-{(2'-(N-triphenylmethyltetrazol-5-yl)biphenyl-4-yl)-methyl}-1H-imidazol-5-yl]-2-propensäureethylester

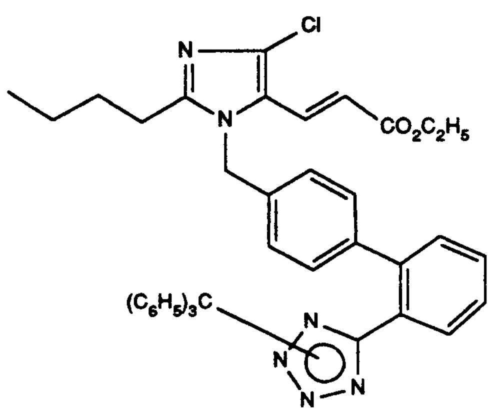

Unter Schutzgas werden 42 mg (1,2 mmol) Natriumhydrid (80%ig) in 6 ml THF suspendiert, 224 mg (1,0 mmol) Phosphonoessigsäuretriethylester bei 25 °C zugetropft, anschließend 1 h bei 25 °C gerührt und 460 mg (0,69 mmol) 2-n-Butyl-4-chlor-1-[2'-(N-triphenylmethyltetrazol-5-yl)biphenyl-4-yl-methyl]-1H-imidazol-5-carboxaldehyd in 4 ml THF zugetropft. Es wird 20 h bei 25 °C nachgerührt. Nach Einengen wird der Rückstand zwischen Wasser/Essigester verteilt, die organische Phase über Natriumsulfat getrocknet, eingeengt und der Rückstand über Kieselgel 60 mit Essigester/Petrolether(1:1) chromatographiert.

Ausbeute: 400 mg (79% der Theorie)
$R_f$ = 0,65 (Essigester/Petrolether = 1:1)

Beispiel III

(E)-3-[2-n-Butyl-1-{(2'-(-N-triphenylmethyltetrazol-5-yl)biphenylyl-4-yl)-methyl}-1H-imidazol-5-yl]-2-propensäureethylester

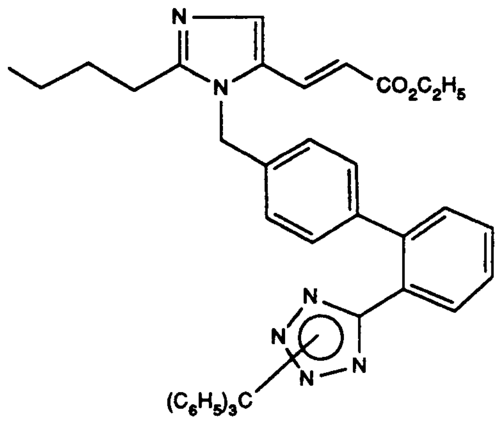

In Analogie zur Vorschrift des Beispiels II wurde die Titelverbindung aus 1,8 g (2,86mmol) der Verbindung aus Beispiel I hergestellt.
Ausbeute: 1,31 g (66 % der Theorie)
$R_f$ = 0,37 (Essigester/Petrolether = 1:1)

Herstellungsbeispiele

Beispiel 1

(E)-3-[2-n-Butyl-4-chlor-1-{(2'-(tetrazol-5-yl)biphenyl-4-yl)methyl}-1H-imidazol-5-yl]-2-propensäure-ethylester

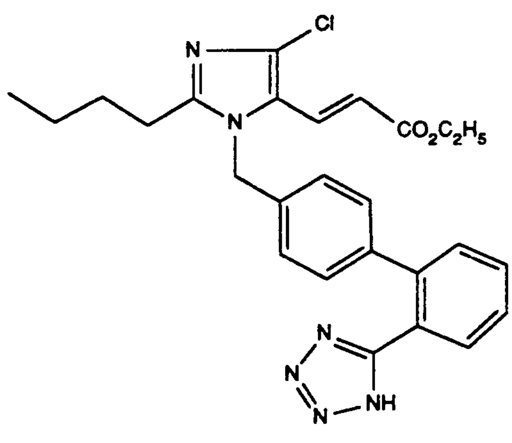

122 mg (0,166 mmol) der Verbindung aus Beispiel II werden in 3,5 ml Eisessig gelöst. Anschließend werden 3,5 ml Wasser zugegeben. Es wird 2 h bei 80 ° C und 20 h bei 25 ° C gerührt. Nach Einengen wird der Rückstand über Kieselgel 60 mit Dichlormethan/Methanol (8:1) chromatographiert.
Ausbeute: 52,4mg (64% der Theorie)
$R_f$ = 0,55 (Essigester: Methanol = 4:1)

Beispiel 2

(E)-3-[2-n-Butyl-4-chlor-1-{(2'-(tetrazol-5-yl)biphenyl-4-yl)methyl}-1H-imidazol-5-yl]-2-propensäure

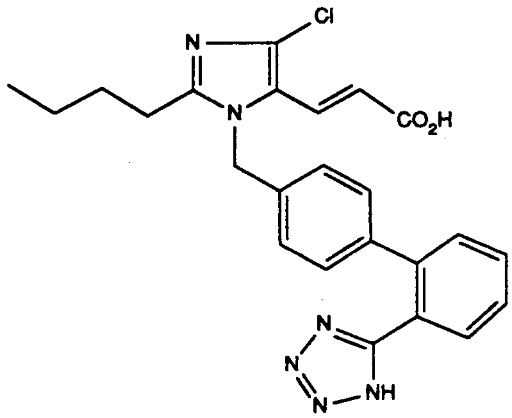

235 mg (0,32 mmol) der Verbindung aus Beispiel 1 werden in 12 ml 1 N methanolischer Natronlauge gelöst, 30 min zum Sieden erhitzt und 1 h bei 25 °C gerührt. Es wird mit konz. Salzsäure auf pH 1 angesäuert, 10 ml Essigester zugegeben und 15 min intensiv gerührt. Die organische Phase wird über Natriumsulfat getrocknet, eingeengt und der Rückstand über Kieselgel 60 mit Essigester / Methanol / Ammoniak = 3:2:0,02 chromatographiert.
Ausbeute: 87 mg (59% der Theorie)
$R_f$ = 0,33 (Essigester/Methanol/Ammoniak = 2:1:0,06)

Beispiel 3

(E)-3-[2-n-Butyl-1-{(2'-(tetrazol-5-yl)biphenyl-4-yl)methyl}-1H-imidazol-5-yl]-2-propensäure

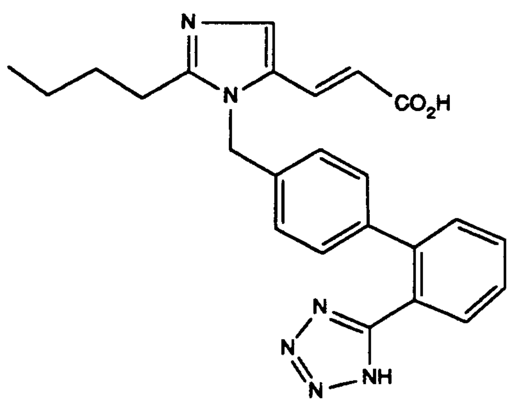

Zu einer Lösung von 1,3 g (1,86 mmol) der Verbindung aus Beispiel III in 25 ml THF werden nacheinander 5 ml Wasser und 5 ml Trifluoressigsäure gegeben. Anschließend wird 16 h bei 25 °C gerührt, mit konz. Natronlauge auf pH 14 gestellt, mit Ether extrahiert, die wäßrige Phase mit halbkonz. Salzsäure angesäuert und dreimal mit je 20 ml Essigester extrahiert. Die organische Phase wird über Natriumsulfat getrocknet, eingeengt und der Rückstand über Kieselgel 60 mit Dichlormethan/Methanol (5:1) chromatographiert.
Ausbeute: 150mg (19% der Theorie)
$R_f$ = 0,18 (Dichlormethan/Methanol = 5:1).

## Patentansprüche

1. Imidazolyl-propensäurederivate der allgemeinen Formel

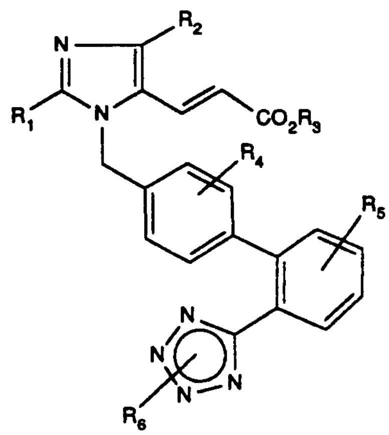

(I),

in welcher

| | |
|---|---|
| $R_1$ | für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder<br>für Cycloalkyl mit 3 bis 8 Kohlenstoffatomen steht, |
| $R_2$ | für Wasserstoff, Halogen, Hydroxy, Nitro, Cyano, Trifluormethyl, Trifluormethoxy oder für Pentafluorethyl steht, oder für geradkettiges oder verzweigtes Alkyl mit bis zu 6 Kohlenstoffatomen steht, oder<br>für Aryl mit 6 bis 10 Kohlenstoffatomen steht, |
| $R_3$ | für Wasserstoff, geradkettiges oder verzweigtes Alkyl mit bis zu 8 Kohlenstoffatomen oder Phenyl steht, |
| $R_4$ und $R_5$ | gleich oder verschieden sind und<br>für Wasserstoff, Halogen, Cyano, Nitro, Trifluormethyl, Hydroxy, Trifluormethoxy oder für geradkettiges oder verzweigtes Alkyl oder Alkoxy mit jeweils bis zu 6 Kohlenstoffatomen stehen, |
| $R_6$ | für Wasserstoff oder für geradkettiges oder verzweigtes Alkyl mit bis zu 6 Kohlenstoffatomen steht |

und deren Salze.

2. Imidazolyl-Propensäurederivate nach Anspruch 1 wobei

| | |
|---|---|
| $R_1$ | für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind,<br>für Cyclopropyl, Cyclopentyl oder Cyclohexyl steht, |
| $R_2$ | für Wasserstoff, Fluor, Chlor, Brom, Iod, Trifluormethyl, Trifluormethoxy, Pentafluorethyl, Phenyl oder für geradkettiges oder verzweigtes Alkyl mit bis zu 6 Kohlenstoffatomen steht, |
| $R_3$ | für Wasserstoff oder für geradkettiges oder verzweigtes Alkyl mit bis zu 6 Kohlenstoffatomen steht, |
| $R_4$ und $R_5$ | gleich oder verschieden sind und<br>für Wasserstoff, Fluor, Chlor, Brom, Trifluormethyl, Trifluormethoxy oder für geradkettiges oder verzweigtes Alkyl mit bis zu 4 Kohlenstoffatomen stehen, |
| $R_6$ | für Wasserstoff oder für geradkettiges oder verzweigtes Alkyl mit bis zu 4 Kohlenstoffatomen steht |

und deren Salze.

3. Imidazolyl-propensäurederivate nach Anspruch 1, wobei

| | |
|---|---|
| $R_1$ | für geradkettiges oder verzweigtes Alkyl oder Alkenyl mit jeweils bis zu 4 Kohlenstoff- |

atomen oder Cyclopropyl steht,

$R_2$ für Wasserstoff, Fluor, Chlor, Brom, Jod, Trifluormethyl, Pentafluorethyl, Phenyl oder für geradkettiges oder verzweigtes Alkyl mit bis zu 4 Kohlenstoffatomen steht,

$R_3$ für Wasserstoff oder für geradkettiges oder verzweigtes Alkyl mit bis zu 4 Kohlenstoffatomen steht,

$R_4$ und $R_5$ gleich oder verschieden sind und für Wasserstoff, Fluor, Chlor oder Brom stehen,

$R_6$ für Wasserstoff, Methyl, Ethyl, Isopropyl oder Propyl steht

und deren Salze.

**4.** Imidazolyl-propensäurederivate nach Anspruch 1, wobei

$R_1$ für geradkettiges oder verzweigtes Alkyl oder Alkenyl mit jeweils bis zu 4 Kohlenstoffatomen steht,

$R_2$ für Wasserstoff, Fluor, Chlor, Iod, Trifluormethyl oder Pentafluorethyl steht,

$R_3$ für Wasserstoff, Methyl oder Ethyl steht

und

$R_4$, $R_5$ und $R_6$ für Wasserstoff stehen

und deren Salze.

**5.** Imidazolyl-propensäurederivate nach Anspruch 1 zur Bekämpfung von Krankheiten.

**6.** Verfahren zur Herstellung von Imidazolyl-propensäurederivaten der allgemeinen Formel

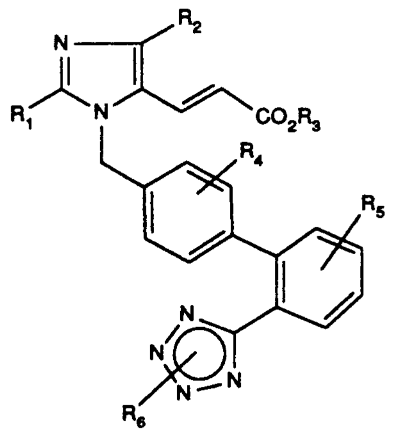

(I),

in welcher

$R_1$ für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder für Cycloalkyl mit 3 bis 8 Kohlenstoffatomen steht,

$R_2$ für Wasserstoff, Halogen, Hydroxy, Nitro, Cyano, Trifluormethyl, Trifluormethoxy oder für Pentafluorethyl steht, oder für geradkettiges oder verzweigtes Alkyl mit bis zu 6 Kohlenstoffatomen steht, oder für Aryl mit 6 bis 10 Kohlenstoffatomen steht,

$R_3$ für Wasserstoff, geradkettiges oder verzweigtes Alkyl mit bis zu 8 Kohlenstoffatomen oder Phenyl steht,

$R_4$ und $R_5$ gleich oder verschieden sind und für Wasserstoff, Halogen, Cyano, Nitro, Trifluormethyl, Hydroxy, Trifluormethoxy oder für geradkettiges oder verzweigtes Alkyl oder Alkoxy mit jeweils bis zu 6 Kohlenstoffatomen stehen,

$R_6$ für Wasserstoff oder für geradkettiges oder verzweigtes Alkyl mit bis zu 6 Kohlenstoffatomen steht

und deren Salze,

dadurch gekennzeichnet, daß man Aldehyde der allgemeinen Formel (II)

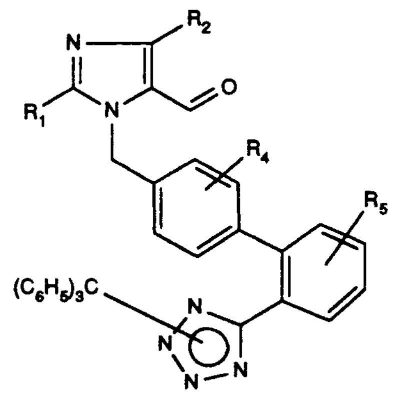

(II),

in welcher

$R_1$, $R_2$, $R_4$ und $R_5$ die oben angegebene Bedeutung haben,

mit Phosphonoessigsäureestern der allgemeinen Formel (III)

$(R^7O)_2$-P(O)-$CH_2$-$CO_2R^7$ (III),

in welcher
$R_7$ die oben angegebene Bedeutung von $R_3$ hat, aber nicht für Wasserstoff steht,
in inerten Lösemitteln, in Anwesenheit einer Base zu den Verbindungen der allgemeinen Formel (IV)

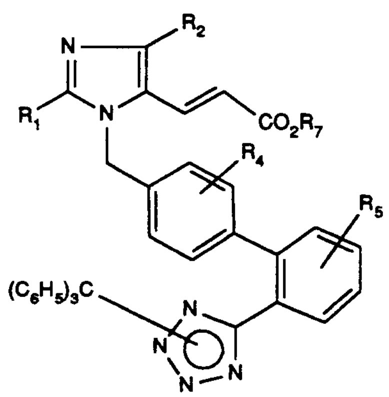

(IV),

in welcher

$R_1$, $R_2$, $R_4$, $R_5$ und $R_7$ die oben angegebene Bedeutung haben,

umsetzt und anschließend die Tritylgruppe mit Säuren abspaltet,

und im Fall der Säuren($R_3$ = H) die Ester verseift

und im Fall, daß $R_6$ nicht für Wasserstoff steht, alkyliert.

**7.** Verfahren nach Anspruch 6 dadurch gekennzeichnet, daß man die Umsetzung in einem Temperaturbereich von -30 ° C bis + 100 ° C durchführt.

**8.** Arzneimittel enthaltend mindestens ein Imidazolylpropensäurederivat nach Anspruch 1.

**9.** Arzneimittel nach Anspruch 8 zur Behandlung von Bluthochdruck und Atherosklerose.

**10.** Verwendung von Imidazolyl-propensäurederivaten zur Herstellung von Arzneimitteln.

Europäisches Patentamt

# EUROPÄISCHER RECHERCHENBERICHT

Nummer der Anmeldung

EP 92 11 5970

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP-A-0 403 158 (SMITH BEECHAM CORPORATION)<br>--- | | C07D403/10<br>A61K31/415<br>A61K31/41 |
| D,A | EP-A-0 403 159 (SMITH BEECHAM CORPORATION)<br>----- | | |
| | | | **RECHERCHIERTE SACHGEBIETE (Int. Cl.5)** |
| | | | C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 22 DEZEMBER 1992 | DE BUYSER I.A.F. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)